# EUROPEAN PATENT SPECIFICATION

(11) **EP 2 001 671 B1**
(45) Date of publication and mention of the grant of the patent: **19.05.2010**
(21) Application number: 07711294.4
(22) Date of filing: 28.03.2007
(51) Int. Cl.: B32B 27/08

(54) **A MULTILAYER FILM FREE OF HALOGENS WHICH IS IMPERMEABLE TO AROMA COMPOUNDS**
FÜR AROMAVERBINDUNGEN UNDURCHLÄSSIGE, HALOGENFREIE MEHRSCHICHTFOLIE
FILM MULTICOUCHE EXEMPT D'HALOGÈNES ET IMPERMÉABLE AUX COMPOSÉS AROMATIQUES

(30) Priority: 28.03.2006 DK 200600443; 28.03.2006 US 786649 P
(43) Date of publication of application: 17.12.2008
(73) Proprietor: Amcor Flexibles Transpac B.V.B.A., 1935 Zaventem (BE)
(72) Inventor: RASMUSSEN, Torben, DK-7100 Vejle (DK); SLØK, Michael, DK-6670 Holsted (DK)
(74) Representative: Jensen, Peter Kim
(86) International application number: PCT/DK2007/000153
(87) International publication number: WO 2007/110080

(56) References cited:
- EP-A- 0 370 755
- EP-A- 0 433 060
- EP-A- 0 700 777
- EP-A- 0 958 916
- EP-A- 1 422 057
- EP-A- 1 598 180
- EP-A2- 0 603 680
- WO-A-01/00408
- WO-A-01/00716
- WO-A-96/40429
- WO-A-03/055678
- US-A- 5 567 489
- DATABASE WPI Week 198719 Derwent Publications Ltd., London, GB; AN 1987-133385 XP002432627 & JP 62 074646 A (TORAY IND INC) 6 April 1987 (1987-04-06)

## Description

### Technical Field

The present invention relates to a multilayer film free of halogens which is impermeable to aroma compounds, comprising at least one barrier layer and at least one skin layer, such as a film, to be used in the production of ostomy pouches.

### Background

Multilayer films free of halogens which are impermeable to aroma compounds are in particular useful for the production of pouches, such as ostomy pouches, drainable bags, and the like pouches. When using a multilayer film for making an ostomy pouch, it is very important that urine, excrements, and flatus gases do not penetrate the film before the pouch is disposed of. The ostomy pouch may be provided with a filter for letting the flatus gases out to avoid balloon-like inflation of the pouch. The filter may contain a special carbon solution neutralizing odour in order to neutralise the odour of the flatus gases.

Multilayer films for the manufacturing of ostomy pouches, drainage pouches, or the like pouches are made of polymers comprising halogens, such as polyvinylchloride, also known as PVC, or polyvinylidenechloride, also known as PVdC, for obtaining a sufficient gas and fluid barrier. However, such halogen-containing products have been found to have a serious environmental impact when disposed of. Pouches are as such disposable items affecting the environment, and when the pouches further contain halogens also affecting the environment, the negative effect on the environment is heightened. Thus, there is a need of a multilayer film free of halogen.

By a multilayer film is meant a film having more than one layer, i.e. at least one barrier layer and one other layer different from the aroma barrier layer, such as a skin layer. Thus, the multilayer film may comprise other layers which have merely been at least partly adhered or sealed to the rest of the rest of the film. A skin layer according to the invention is defined as the outermost layer of the multilayer film.

Previous studies have indicated that compounds responsible for faecal odour are mainly indoles and sulphide derivatives. In order to test the aroma barrier properties in a barrier layer, tests are usually carried out by testing the break through time for penetration of 3-Methyl indole (skatole) and/or dimethyl disulphide (DMDS) through the barrier layer often incorporated in a multilayer film.

In addition to aroma barrier properties in ostomy or incontinence applications, it is desirable that polymeric films do not emit noise when flexes, is oxygen and water vapour impermeable in a certain time period, is comfortable to wear close to the skin of the user of ostomy or incontinence applications, and has a high puncture resistance, good heat seating properties, and a good flex crack resistance.

Film structures free of halogens is known from US 6,455,161, US 5,567,489, and EP 1 598 180 A1, wherein the barrier layer is based on PET-G or Nylon,

Further WO 01/00716 A2 being the closest prior art discloses a multilayer film free of halogens and having an aroma barrier layer comprising thermoplastic polyurethane and a sealable skin layer.

### Description of the Invention

An object of the present invention is, at least partly, to overcome the disadvantages of prior art and provide an alternative multilayer film free of halogens having the same or better properties as the known films free of halogens for the manufacture of products and having improved properties, e.g. a good barrier towards aroma compounds, such as skatole (3-Methyl indole) and Dimethyl disulphide (DMDS), less stiffness, a more rubber-like surface and touch, good tensile strength, good flex crack resistance, good puncture resistance, reduction of noise when the multilayer film its flexed, and/or sufficient oxygen and water vapour impermeability.

This object and the advantages becoming evident from the description below are obtained by a multilayer film free of halogens which is impermeable to aroma compounds and having the features stated in claim 1.

By "impermeable to aroma compounds" is meant that a multilayer film comprising an aroma barrier layer having the thickness of 15 µm or less is impermeable to skatole in at least 4 hours, meaning that no penetration of skatole through the multilayer film occurs when the multilayer film is subjected to an aroma test, such as a skatole test. The aroma test or the skatole test is performed according to a British Standard, BS 7127, part 101 (1991) at 40 °C. REMARKS: The film is sealed into a test pouch with a fixed pre-selected area which is filled with a saturated skatole solution and then hermetically sealed. Subsequently the film pouch is placed in an aluminium based bag being substantially aroma impermeable and the bag is then filled with a fixed, specified amount of demineralised water. Finally, the aluminium bag is hermetically sealed) and kept at 40°C. The existence of skatole compound in the air surrounding the pouch in the bag is then determined after 4 hours and each subsequent hour after this until a significant increase of skatole is detected. The test is performed by a test panel of at least 3 persons

The advantage of having a barrier layer comprising thermoplastic polyurethane is that the barrier layer can be very thin, i.e. thinner than barrier layers in known multilayer films, while still being impermeable towards aroma compounds, such as skatole, and still keeping high mechanical strength. In the production of ostomy pouches or the like products, it is of great importance that the barrier layer is as thin as possible since a thin barrier layer results in a less stiff multilayer film and thereby a less stiff pouch which is more comfortable to wear. Furthermore, a thin barrier layer results in a multilayer film making less noise when flexed, e.g. when blended in several directions or even twisted.

In particular thermoplastic polyurethane can be made into a very thin aroma barrier layer, such as 10 µm or less in the result of this is a multilayer film which has a very low stiffness and which thus makes less noise than films comprising other polymers applicable as barrier layer, e.g. polyamide, since polyamide does not have as excellent barrier properties as thermoplastic polyurethane in the same thickness.

In this way, alternative multilayer films free of halogens and impermeable to aroma compounds have been obtained.

The aroma barrier layer comprises more than 40 weight% thermoplastic polyurethane polymer.

The thermoplastic polyurethane polymer has an E-modulus of at least 600 MPa, at least 1000 MPa, at least 1700 MPa, or at least 2000 MPa.

In addition, the thermoplastic polyurethane polymer of the aroma barrier layer may further comprise a blend with other polymers, such as other polyurethanes or polyester.

Furthermore, the multilayer film may comprise a sulphur absorber. The sulphur absorber may also be comprised in the skin layer and/or the barrier layer and/or an intermediate layer.

In another embodiment, the skin layer may have an E-modulus that is less than 100 MPa or 15,000 Psi.

According to another embodiment of the present invention the multilayer film may comprise at least one skin layer comprising or including:
- styrene-based copolymers, such as styrene ethylene butylene styrene copolymer (SEBS), styrene ethylene propylene styrene copolymer (SEPS), styrene butadiene styrene copolymer (SBS), or styrene isoprene styrene copolymer (SIS):
- ethylone-based polymers, such as pure polyethylene in the form of low density polyethylene (LDPE), linear low density polyethylene (LLDPE), ultra low density polyethylene (ULDPE), medium density polyethylene (MDPE), high density polyethylene (HOPE), or cyclic polyolefin (COC);
- ethylene-based co-polymers, such as ethylene methyl acrylate copolymer (EMA), ethylene vinyl acetate copolymer (EVA), ethylene butyl acrylate copolymer (EBA), ethylene-ethyl acrylate (EEA), methylene acrylic acid copolymer (EAA), ionomer resins, elastomeric co-polyesters, ethylene-methyl acrylic acid copolymers (EMAA), EVA-carbon monoxide copolymers (EVACO), MAH-modified polyethylene, maleic anhydride modified EVA, MAH-EMA, MAH-EBA, MAH-PP, glycidyl methacrylate modified EMA, glycidyl methacrylate modified EBA, glycidyl methacrylate modified EVA, ethylene butylene copolymer, ethylene 4-methyl pentene copolymer, ethylene hexene co-polymer, ethylene octene copolymer, ethylene propylene copolymer, or ethylene butylene propylene ter-polymer;
- pure polypropylene, pure polybutylene, thermoplastic polyurethane (TPU), homogeneous EAO copolymers, PP homo- or copolymers, rubber modified PP, low modulus PP homo- or copolymers, low crystallinity PP homo- or copolymers, syndiotactic PP homo- or copolymers, ethylene-propylene-diene monomer elastomer (EPDM), ethylene-polypropylene rubbers (EPR), substantially linear EAO copolymers, ethylene-alkyl acrylate copolymers, such as, for example, polynorbornene, ESI, polyether-amide block copolymers, polyamide (PA) or polyester (PETP); or
- any kind of blends of the above.
All mentioned polymers or blends can further be anhydride modified.

By having an aroma barrier layer based of TPU, a multilayer film having only three layers can be made since the skin layer may be made of a polymer compatible with TPU. In this way, a tie layer or bonding layer is dispensable.

A multilayer film having a barrier layer of TPU may be extruded into a multilayer film with a skin layer of soft polyurethane polymers. In this way, a multilayer film with good barrier properties is obtained which only has three layers. Such a three layer film is simple to manufacture and more friendly to the environment since a multilayer film impermeable to aroma compounds can thus be made comprising of at least 95% of the same polymer.

When using thermoplastic polyurethane as an aroma barrier layer, the multilayer film becomes relatively rigid and noisy. Therefore, a skin layer is added to the barrier layer which, in addition to having excellent sealing properties, makes the multilayer film softer and less stiff, and thus also less noisy. A skin layer made from the above-mentioned polymers thus provides sufficient softness and lowers the noise level of the film without resulting in an unacceptable increase in the total thickness of the multilayer film. Furthermore, such a skin layer provides an improved puncture resistance and flex crack resistance as well as better sealing properties, e.g. when heat sealed into a pouch or the like appliances.

Additionally, by providing a multilayer film comprising a thin aroma barrier layer of pure TPU together with skin layers made of e.g. SEBS, the multilayer film may be produced so thin as to be able to be used for the production of ostomy and drainage pouches. The surface of the multilayer film according to the invention is thus very soft, has a low noise level when flexed, has a nice rubber-like and silky feel, and has a good puncture resistance.

In addition, the skin layer may be embossed, texturised, foamed, non-woven, have been subjected to a finishing treatment such as pulling of threads, or have a silky surface.

Furthermore, the aroma barrier layer may be positioned in between two skin layers.

Also, the sulphur absorber may be comprised in the skin layer positioned furthest from the aroma compounds so that the aroma compounds have to enter the barrier layer before arriving at the skin layer containing sulphur absorber.

By having the sulphur absorber in the multilayer film, preferably in the skin layer positioned furthest from the aroma compounds, the sulphur absorber is not used until the aroma compounds have to pass the barrier layer and therefore have been timely delayed. In this way, the multilayer film is able to detain the aroma compounds even further by use of the sulphur absorber in the outmost skin layer.

In another embodiment of the present invention, the multilayer film may further comprise an additional layer. In e.g. an extrusion process, the additional layer may be in the form of a tie layer used for bonding the barrier layer together with the skin layer. The tie layer is used when the barrier layer and skin layer do not adhere sufficiently to each other. In an adhesive lamination process, the additional layer may be in the form of an adhesive layer, such as a 2-component polyurethane adhesive, a hotmelt, a wax, or the like adhesive.

Furthermore, the additional layer may be positioned between the skin layer and the aroma barrier layer.

According to the invention, the additional layer may be a tie layer, which may comprise or include:
- styrene-based copolymers, such as styrene ethylene butylene styrene copolymer (SEBS), styrene ethylene propylene styrene copolymer (SEPS), styrene butadiene styrene copolymer (SBS), or styrene isoprene styrene copolymer (SIS);
- ethylene-based polymers, such as pure polyethylene in the form of low density polyethylene (LDPE), linear low density polyethylene (LLDPE), ultra low density polyethylene (ULDPE), medium density polyethylene (MDPE), high density polyethylene (HDPE), or cyclic polyolefin (COC);
- ethylene-based co-polymers, such as ethylene methyl acrylate copolymer (EMA), ethylene vinyl acetate copolymer (EVA), ethylene butyl acrylate copolymer (EBA), ethylene-ethyl acrylate (EEA), ethylene acrylic acid copolymer (EAA), ionomer resins, elastomeric co-polyesters, ethylene-methyl acrylic acid copolymers (EMAA), EVA-carbon monoxide copolymers (EVACO), MAH-modified polyethylene, maleic anhydride modified EVA, MAH-EMA, MAH-EBA, MAH-PP, glycidyl methacrylate modified EMA, glycidyl methacrylate modified EBA, glycidyl methacrylate modified EVA, ethylene butylene copolymer, ethylene 4-methyl pentene copolymer, ethylene hexene co-polymer, ethylene octene copolymer, ethylene propylene copolymer, or ethylene butylene propylene ter-polymer;
- pure polypropylene, pure polybutylene, thermoplastic polyurethane (TPU), homogeneous EAO copolymers, PP homo- or copolymers, rubber modified PP, low modulus PP homo- or copolymers, low crystallinity PP homo- or copolymers, syndiotactic PP homo- or copolymers, ethylene-propylene-diene monomer elastomer (EPDM), ethylene-polypropylene rubbers (EPR), substantially linear EAO copolymers, ethylene-alkyl acrylate copolymers, such as, for example, polynorbornene, ESI, polyether-amide block copolymers, polyamide (PA) or polyester (PETP); or
- any kind of blends of the above.
All mentioned polymers or blends can further be anhydride modified.

By providing a tie layer between the skin layer and the barrier layer, stronger bonding between different types of polymers is made possible. The tie layer may be made of a polymer blend having a reactive group, such as an anhydride, for bonding the skin layer and barrier layer together chemically or/and mechanically, e.g. in case the skin layer and the barrier layer are not very linkable. A much more free choice of polymer for the skin layer is thus obtained, meaning that polymers having the best sealable properties and/or noise lowering properties etc. may be chosen instead of polymers linkable to the aroma barrier layer.

According to the present invention, the adhesive layer between the barrier layer and skin layer may be a 2-component polyurethane adhesive, a hotmelt, a wax, or the like adhesive.

Additionally, the multilayer film of the present invention may further comprise a non-woven layer. The non-woven layer may be made of polymers, such as polypropylene, polyurethane, polyester or a combination or blend thereof. By adding a non-woven layer to the film, the noise level of the film when flexed, e.g. when exposed to crumpling, is substantially decreased. A result of this is that the thickness of the skin layers of the film may be substantially reduced providing an even thinner total thickness of the pouch which will thus be less visible when worn by the user. By providing a non-woven layer in the multilayer film, layers based on noisier types of polymers having other excellent properties, such as lower cost and better process ability, may also be used.

Furthermore, according to the present invention, the non-woven layer may be laminated or bonded directly to the aroma barrier layer, the multilayer film thus comprising an aroma barrier layer having a non-woven layer on one side and a skin layer on the other side.

According to another embodiment of the present invention, a further non-woven layer may be laminated to the skin layer.

In addition, the hon-woven layer may bye partly heat sealed to the aroma barrier or one of the skin layers.

According to the invention, the sulphur absorber may be coated onto the non-woven layer.

Furthermore, the aroma barrier layer, skin layer; additional layer, and/or non-woven layer may be anhydride modified.

In addition, the aroma barrier layer may have a thickness of 1 - 50 µm, 2 - 25 µm, or 3 - 10 µm.

Also, the skin layer may have a thickness of 5 - 150 µm, 10 - 100 µm, or 15 - 50 µm.

In another embodiment, the multilayer film free of halogens which is impermeable to aroma compounds, may comprise
- at least one aroma barrier layer, and
- at least one other layer positioned furthest from the aroma compounds so that the aroma compounds have to enter the barrier layer before arriving at the other layer, wherein the other layer comprises a sulphur absorber. In one embodiment, the other layer may be a skin layer or a non-woven layer, and in another embodiment, the other layer may be an intermediate layer between the aroma barrier layer and a skin layer.

Finally, the present invention further relates to a pouch, such as an ostomy or drainage pouch manufactured using the multilayer film of the present invention.

### Brief Description of the Drawing(s)

The invention is explained in detail below with reference to the drawings, in which
Fig. 1 shows a sectional view of an embodiment of a multilayer film of the present invention having three layers, and
Fig. 2 shows a sectional view of another embodiment of a multilayer film in accordance with the invention having five layers, and
Fig. 3 shows a sectional view of an embodiment of a multilayer film of the present invention having seven layers

### Detailed description of the preferred embodiments

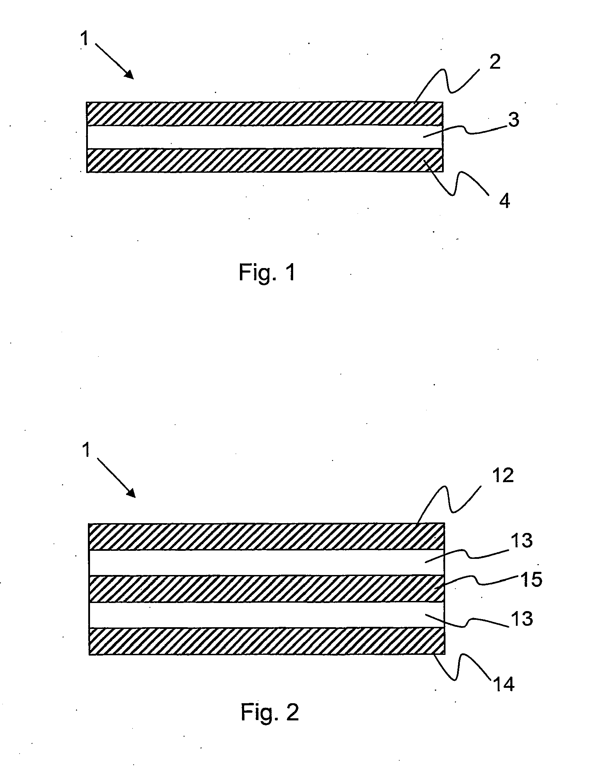

The multilayer film 1 according to the present invention has at least one aroma barrier layer 3 and at least one skin layer 2, 4, and the multilayer film 1 may comprise further layers in a random order as will be disclosed below. In Fig. 1 a, multilayer film is shown having three layers two of which are skin layers 2, 4 and one of which is an intermediate aroma barrier layer 3. The aroma barrier layer 3 has the barrier property of being impermeable to aroma compounds, such as skatole (3-methylindole), in a period of at least 4 hours when the multilayer film 1 is subjected to the above-mentioned skatole test. The skin layers 2, 4 reduce the noise of the multilayer film 1 when the film is crumpled, bended, or wrinkled and may in addition give the surface of the multilayer film 1 a more rubber-like or silky feel. Moreover, the skin layers 2, 4 have excellent sealing properties when the multilayer film 1 is made into a pouch, such as an ostomy or drainage pouch, or is sealed for other purposes.

Even though the multilayer film 1 of Fig. 1 is shown as having two skin layers 2, 4, the multilayer film may, in another embodiment, have only one skin layer. Additionally, the multilayer film of the present invention may comprise one skin layer 2 made of one polymer or a polymer blend, and another skin layer 4 made of another polymer or polymer blend.

The multilayer film 1 of the present invention is particularly useful for ostomy bags (colostomy, ileostomy), trans-dermal delivery systems (TDDS), cosmetic patches, incontinence bags, medical collection bags, and parenteral solution bags. However, the multilayer film 1 of the present invention may also be used for other purposes, such as packaging or wrapping foodstuff or the like where it is of special importance that gas, moisture, and/or aroma compounds are not allowed to come into contact with the foodstuff, or that the wrapped foodstuff does not emit unpleasant odours in the form of aroma compounds. Further, the film 1 may be used for protective clothing applications or soil fumigation.

In Fig. 2 a, multilayer film 1 is shown having two skin layers 12, 14 and an intermediate barrier layer 15. In this embodiment, these layers are co-extruded having tie layers 13.

In another embodiment, the skin layers 12, 14 and the barrier layer 15 are adhesive laminated together by means of polyurethane adhesive bonding layers 13.

The tie layer 13 and the adhesive bonding layer 13 are illustrated as an additional layer 13 in Fig. 2.

Even though the multilayer film 1 of Fig. 2 is shown as having two skin layers 12, 14, the multilayer film may, in another embodiment; have only one skin layer 12. On the other side of the aroma barrier layer, the multilayer film may be provided with another layer, such as a soft non-woven layer 16.

In order to reduce the noise even further, the multilayer film as shown in Fig. 1, having two skin layers 2, 4 and an intermediate aroma barrier layer 3, may also be provided with a non-woven layer.

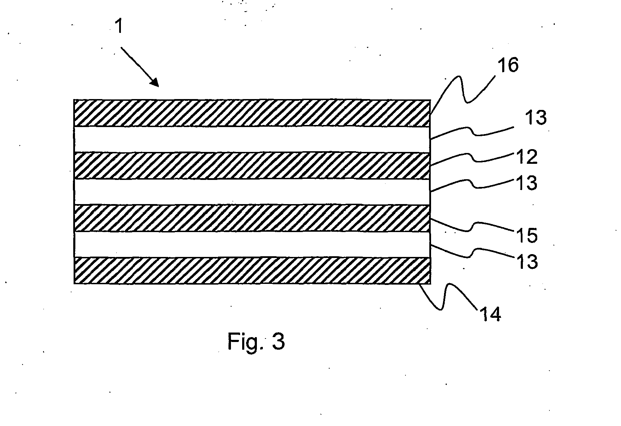

In another embodiment, the soft non-woven layer 16 may be attached to the multilayer film 1 of Fig. 2 by means of a bonding layer as shown in Fig. 3.

In this regard, it should be mentioned that the polymer material of the skin layer and the barrier layer are not always very linkable, resulting in a need of a bonding layer 13 in the form of an adhesive layer or a tie layer. The polymer material of the skin layer 2, 4, 12, 14 is primarily chosen in order for the polymer material to have excellent sealing properties and to be soft enough to damp the noise of the aroma barrier layer when the multilayer film 1 is flexed. The polymer material of the aroma barrier layer 3 is primarily chosen in order for the polymer material to have excellent barrier properties towards aroma compounds, such as skatole, and maybe other excellent barrier properties as well. e.g. towards oxygen, moisture, etc.

The aroma barrier layer 3; 15 of the multilayer film 1 of the present invention comprises or includes TPU.

The film 1 of the present invention may be manufactured by means of production processes such as an adhesive lamination process, an extrusion lamination process, a cast- or blown (co-)extrusion process, and/or an extrusion coating process, or combinations thereof.

The skin layers 2, 4; 12, 14 must be soft and thereby possess excellent noise dampening properties, and must furthermore have excellent seating properties so that the multilayer film can be processed into e.g. a pouch or the like appliances.

According to yet another embodiment the multilayer film 1 of the present invention, the film comprises a tie layer 13 positioned between the skin layers 2, 4, 12, 14 and the barrier layer 3; 15. The function of the tie layer 13 is to bind the barrier layer and the skin layer together in case the polymer materials chosen for the barrier layer 3 and the skin layer 2, 4, 12, 14 are not directly linkable.

According to the invention, the additional layer 13 may be any kind of adhesive layer or tie layer.

The above-mentioned TPU may be a TPU such as an Estane X-4995 Nat021/30 from Noveon,

This TPU is polyester-based, but in another embodiment, the TPU may be polysther-based.

As mentioned, the barrier layer 3 may be made of TPU, such as an Estane X-4995 Nat021/30 from Noveon having an E-modulus of 2130 MPa. The thermoplastic polyurethane useful as a barrier layer has an E-modulus of at least 600 MPa, at least 1000 MPa, at least 1700 MPa, or at least 2000 MPa.

Such stiff thermoplastic polyurethane has a polymeric architecture in which the polymeric chains are positioned closely together, thus providing the wanted advantage of enhanced steric hindrance or steric resistance towards aroma compounds such as mainly indoles and sulphide derivatives such as DMDS and skatole, thus constituting an aroma barrier 3; 15 toward the above-mentioned aroma compounds.

Soft thermoplastic polyurethane has a similar polymeric architecture, but do not have the advantage of enhanced steric hindrance or steric resistance towards aroma compounds as the position of the polymer chains is different.

Since the multilayer film 1 is particularly suitable for the production of ostomy pouches, the film is, in one embodiment, made with a softening layer of a non-woven film. This non-woven layer 16 of film reduces the noise of the stiff material of the barrier layer 3 substantially, and the thickness of the skin layers 12, 14 may therefore be substantially reduced when such a non-woven layer 16 is applied. A non-woven layer 16 in the multilayer film 1 further gives the possibility of using skin layers based on noisier types of polymers, but with other advantageous properties, e.g. lower cost, better process ability, etc.

The multilayer film of the present invention has a barrier layer 3; 15 in the thickness of 1 - 50 µm, such as 2 - 25 µm, or such as 3 - 10 µm. The skin layer is manufactured witch a thickness of 5 - 150 µm, such as 10 -100 µm, or such as 15 - 50 µm. When the multilayer film further comprises a non-woven layer 16, the thickness of the skin layer can be reduced to 5 - 75 µm, such as 5 - 50 µm, or such as 5 - 25 µm, since the non-woven layer reduces the noise substantially.

Furthermore, the multilayer film of the present invention may contain one or more of the following additives: processing aids, such as siloxanes, silicones or fluoropolymers; inorganic fillers such as calcium carbonate, barium sulphate, mica, silica, silica gel, nano-fillers and talc; slip additives such as fatty acid amides; antiblock additives; odour absorbers; humidity absorbers; molecular sieves; pigments; antistatic additives; antifog agents; antioxidants; UV stabilizers; dielectric heating sensitizing additives; pigments; colours; activated carbon; fragrance; nucleating agents, clarifiers; biocides; and antimicrobial additives.

The multilayer film may, at least on one outside layer, be subjected to a surface treatment such as corona treatment, flame treatment, or plasma treatment in order to increase its surface tension and improve its printability.

Furthermore, one surface of the film may be vacuum-coated with a thin layer of metal or metal oxide such as aluminium, aluminium oxide, or silicium oxide.

In accordance with the present invention, the non-chlorinated polymeric films are used as barriers to odours and organic molecules. The polymeric films may be used as single or monolayer films or as component films of multilayer film structures. Examples of such multilayer film structures may comprise, but are not limited to, 2 to 7 layers and could, for example, take the form of A/B/D/C/D/E/F or A/B/C/B/A or A/B/C/D/E or A/B/C/D, or A/C/B/, or C/B, with the "C" layer being the essential barrier layer in the non-chlorinated film layer of the present invention and the other layers comprising adhesive, intermediate, or skin layers.

A skin layer according to the invention is defined as the outermost layer of the multilayer film and as soft and therefore a noise dampening layer. By soft is meant a layer of a polymer having an E-modulus less than 100 MPa or 15000 Psi.

In the following, examples multilayer films have been tested by use of different tests:

### EXAMPLE 1

A five layer film as shown in Fig. 2, having skin layers 12, 14 based on SEBS (GRP6571, Kraton), intermediate tie layers 13 of an anhydride modified styrene-based co-polymer (Kraton FG 1901) or anhydride-modified ethylene copolymer (Bynel 3861, DuPont), and a barrier layer 15 of TPU (Estane X-4995 Nat021/30, Noveon) was blown co-extruded according to the present invention. The film was manufactured with an aroma barrier layer in the thickness of 3 µm (film A2, B2, C2, E2 of Table 1) and with an aroma barrier layer in the thickness of 5 µm (film D2 of Table 1), and skin layers of the different thickness as can be seen in Table 1. All combinations shown in Table 1 were subsequently tested according to the above-mentioned skatole test, and no skatole penetration was observed after 24 hours as can be seen in Table 1.

**Table 1**

| Film No. | Thickness [µm] SEBS GRP6571 Kraton | Thickness [µm] Tie layer FG 1901 Kraton / Bynel 3861 DuPont | Thickness of barrier layer [µm] TPU Estane X-4995 Noveon | Thickness [µm] Tie layer FG 1901 Kraton / Bynel 3861 DuPont | Thickness [µm] SEBS GRP6571 Kraton | Time for Skatole Penetration [hours] |
|---|---|---|---|---|---|---|
| A2 | 18 | 3 (3861) | 3 | 3 (3861) | 18 | > 24 |
| B2 | 23 | 3 (3861) | 3 | 3 (3861) | 23 | |
| C2 | 35 | 3 (1901) | 3 | 3 (1901) | 35 | |
| D2 | 35 | 3 (1901) | 5 | 3 (1901) | 35 | |
| E2 | 45 | 3 (1901) | 3 | 3 (1901) | 45 | |

### EXAMPLE 2

A three layer film as shown in Fig 1, having skin layers 2, 4 based on EMA (Elvaloy 1224 AC, DuPont) and an intermediate barrier layer 3 of PA (SELAR PA 3426, DuPont) was co-extruded according to the present invention. The film was manufactured with an aroma barrier layer in the thickness of 7 µm (film A3 and B3 of Table 2) and another aroma barrier layer in the thickness of 10 µm (C3 and D3 of Table 2), and skin layers of the different thickness as can be seen in Table 2. All combinations shown in Table 2 were subsequently tested according to the above-mentioned skatole test and skatole penetration was observed after 19 hours as can be seen in Table 2.

**Table 2**

| Film No. | Thickness [µm] EMA Elvaloy 1224 AC Dupont | Thickness of barrier layer [µm] PA SELAR 3426, DuPont | Thickness [µm] EMA Elvaloy 1224 AC Dupont | Time for Skatole Penetration [hours] |
|---|---|---|---|---|
| A3 | 35 | 7 | 35 | = 19 |
| B3 | 45 | 7 | 45 | |
| C3 | 35 | 10 | 35 | |
| D3 | 45 | 10 | 45 | |

### EXAMPLE 3

Another multilayer film was made having seven layers, as shown in Fig 3., being film A2 of Example 2 laminated together with a non-woven film layer (grade 25707, Ahlstrom Windsro Locks) 16 by the means of a 2-component polyurethane adhesive (7740/6065, Henkel). The film was subsequently tested according to the above-mentioned skatole test, and no skatole penetration was observed after 24 hours as can be seen in Table 3.

**Table 3**

| Film No. | Thickness [g/m2] Non-woven 25707 Ahlstrom | Thickness [µm] Adhesive 7740/6065 Henkel | Thickness [µm] SEBS GRP6571 Kraton | Thickness [µm] Tie layer Bynel 3861 DuPont | Thickness of barrier layer [µm] TPU Estane X-4995 Noveon | Thickness [µm] Tie layer Bynel 3861 DuPont | Thickness [µm] SEBS GRP6571 Kraton |
|---|---|---|---|---|---|---|---|
| A4 | 22 | 1,8 | 18 | 3 | 3 | 3 | 18 |
| Time for Skatole Penetration > 24 hours | | | | | | | |

### EXAMPLE 4

Another multilayer film was made having 5 layers, as shown in Fig 2, being one skin layer 12 based on SEBS (GRP6571, Kraton), one non-woven layer 14 (grade 25707, Ahlstrom Windsro Locks), and one aroma barrier layer 15 of polyester (Mylar FA, DuPont) adhesive laminated according to the present invention by the means of a 2-component polyurethane adhesive (7740, 6065 Henkel) 13. The multilayer film was subsequently tested according to the above-mentioned skatole test, and no skatole penetration was observed after 24 hours as can be seen in Table 4.

**Table 4**

| Film No. | Thickness [g/m2] Non-woven 25707 Ahlstrom | Thickness [µm] adhesive 7740/6065 Henkel | Thickness of barrier layer [µm] PETP Molar FA Dupont | Thickness [µm] adhesive 7740/6065 Henkel | Thickness [µm] SEBS GRP6571 Kraton | Time for Skatole Penetration [hours] |
|---|---|---|---|---|---|---|
| A5 | 22 | 1,8 | 6 | 1,8 | 45 | > 24 |

In the following examples, namely Example 5 to 16 and table 5 to 16, the following 8 films have been tested:
Film 1: A five layer film as shown in Fig. 2, having skin layers 12, 14 based on SEBS (GRP6571, Kraton), intermediate tie layers 13 of an anhydride-modified ethylene co-polymer (Bynel 3861, DuPont), and a barrier layer 15 of polyester based TPU (Estane X-4995 Nat021/30, Noveon) was blown co-extruded according to the present invention. The film was manufactured with a TPU aroma barrier layer in the thickness of 7 µm, skin layers in the thickness of 32 µm, and tie layers in the thickness of 5 µm.
Film 2: A five layer film as Film 1, wherein the film was manufactured with a TPU aroma barrier layer in the thickness of 10 µm and skin layers in the thickness of 30 µm.
Film 3: A five layer film as Film 1, wherein the skin layer positioned furthest from the aroma compounds further comprises a 15% Masterbatch (SAB) consisting of 85% LDPE (Escorene LD 100 BW, ExxonMobil Chemical) and 15% sulphur absorber (TEGO® Sorb PY 88 T.Q. from Degussa). The relative content of the sulphur absorber is therefore 2.25 % in the skin layer.
Film 4: A seven layer film having skin layers based on SEBS (GRP6571, Kraton), intermediate layers of EVA (Escorene Ultra EVA FL 00226, ExxonMobil Chemical), tie layers of an anhydride-modified ethylene copolymer (Bynel 3861, DuPont), and a TPU barrier layer 15 of TPU (Estane X-4995 Nat021/30, Noveon) was blown co-extruded according to the present invention. The film was manufactured with a TPU aroma barrier layer in the thickness of 10 µm, intermediate layers in the thickness of 10 µm, skin layers in the thickness of 20 µm, and tie layers in the thickness of 5 µm.
Film 5: A three layer film as shown in Fig 1, having skin layers 2, 4 based on EMA (Elvaloy 1224 AC, DuPont) and a barrier layer 3 of PA (SELAR PA 3426, DuPont) was co-extruded according to the present invention. The film was manufactured with a PA aroma barrier layer in the thickness of 7 µm and skin layers in the thickness 35 µm.
Film 6: A four layer film having a 25 µm thick skin layer of EVA (with approx. 17% Vinyl acetate), a 10 µm thick barrier layer 15 of PVDC (having a melting point of 155°C), a 10 µm thick layer of LLDPE (having a melting point of 116°C/122°C), and a 30 µm thick skin layer of EVA (with approx. 20% Vinyl acetate) was co-extruded according to the present invention.
Film 7: A three layer film as shown in Fig 1, having skin layers 2, 4 of LLDPE (EXACT 4151, ExxonMobil Chemical) and a COC barrier layer 3 in a blend of 95% COC (TOPAS 9506F-04, TOPAS Advanced Polymers) and 5% LLDPE (EXACT 4151, ExxonMobil Chemical) was co-extruded according to the present invention. The film was manufactured with a COC aroma barrier layer in the thickness of 10 µm and skin layers in the thickness 15 µm.
Film 8: A three layer film as shown in Fig 1, having skin layers 2, 4 of LLDPE (EXACT 4151, ExxonMobil Chemical) and a COC barrier layer 3 in a blend of 90% COC (TOPAS 9506F-04, TOPAS Advanced Polymers) and 10% LLDPE (EXACT 4151, ExxonMobil Chemical) was co-extruded according to the present invention. The film was manufactured with a COC aroma barrier layer in the thickness of 10 µm and skin layers in the thickness 15 µm.

### EXAMPLE 5

Oxygen permeability (ml O₂/ / (24 hours*m^{2*}atm)) has been tested according to ASTM 3985 at 23°C in the environment of N₂ humid / O₂ Dry.

The thickness of the whole film and that of the barrier layer in the film were measured in a microscope.

**Table 5**

| | Test | Thickness of multi-layer film | Thickness of barrier layer | Oxygen permeability |
|---|---|---|---|---|
| | Test method | Microscope | Microscope | ASTM 3985 |
| | Test conditions | | | 23 °C (N₂ Humid /O₂ Dry) |
| | Unit | [µm] | [µm] | ml O₂ / (24 hours*m²*atm.) |

| Film no. | Composition | | | |
|---|---|---|---|---|
| **1** | 32µm SEBS/5µm TIE/**7**µ**m TPU**/5µm TIE/ 32µm SEBS | 81 | 7 | 1013 |
| **2** | 30µm SEBS/5µm TIE/**10**µ**m TPU**/5µm TIE /30µm SEBS | 80 | 10 | 947 |
| **3** | 32µm (85%SEBS+15%SAB)/5µm TIE/ **7**µ**m TPU**/5µm TIE/32µm SEBS | 81 | 7 | 967 |
| **4** | 20µm SEBS/10µm EVA/5µm TIE/ **10**µ**m TPU**/5µm TIE/10µm EVA/20µm SEBS | 80 | 10 | 963 |
| **5** | 35µm EMA/**7**µ**m PA**/35µm EMA | 77 | 7 | 267 |
| **6** | 25µm EVA/**10**µ**m PVDC**/10µm LLDPE/ 30µm EVA | 75 | 10 | 8,5 |
| **7** | 15µm LLDPE/ **10**µ**m (95%COC+5%LLDPE)/** 15µm LLDPE | 40 | 10 | 3010 |
| **8** | 15µm LLDPE/ **10**µ**m (90%COC+10%LLDPE)/** 15µm LLDPE | 40 | 10 | 2990 |

### EXAMPLE 6

Water vapour permeability (gram H₂O/ (24 hours*m²)) has been tested according to ASTM F1249 at 38°C and a relative humidity (RH) of 90%

**Table 6**

| | Test | Water Vapour permeability |
|---|---|---|
| | Test method | ASTM F1249 |
| | Test conditions | 38 ° C, 90 % RH |
| | Unit | Gram H₂O / (24 hours*m²) |

| Film no. | Composition | |
|---|---|---|
| **1** | 32µm SEBS/5µm TIE/**7**µ**m TPU**/5µm TIE/ 32µm SEBS | 22,4 |
| **2** | 30µm SEBS/5µm TIE/**10**µ**m TPU**/5µm TIE /30µm SEBS | 21,0 |
| **3** | 32µm (85%SEBS+15%SAB)/5µm TIE/ **7**µ**m TPU**/5µm TIE/32µm SEBS | 21,0 |
| **4** | 20µm SEBS/10µm EVA/5µm TIE/ **10**µ**m TPU**/5µm TlE/10µm EVA/20µm SEBS | 32,2 |
| **5** | 35µm EMA/**7**µ**m PA**/35µm EMA | 41,6 |
| **6** | 25µm EVA/**10**µ**m PVDC**/10µm LLDPE/ 30µm EVA | 4,5 |
| **7** | 15µm LLDPE/ **10**µ**m (95%COC+5%LLDPE)/** 15µm LLDPE | 7,0 |
| **8** | 15µm LLDPE/ **10**µ**m (90%COC+10%LLDPE)/** 15µm LLDPE | 6,7 |

### EXAMPLE 7

Seal strength at break (N / inch) has been tested according to ASTM D882 at 23°C and 50% RH. The sealing has been made at a pressure of 0,4 N/mm² at a temperature of 120°C and in 0.5 sec.

**Table 7**

| | Test | Seal Strength at break |
|---|---|---|
| | Test method | ASTM D882 |
| | Test conditions | 23 °C, 50 % RH Sealing: Pressure = 0,4 N/mm² Temperature = 120 °C Time = 0.5 Sec. |
| | Unit | N / Inch |

| Film no. | Composition | |
|---|---|---|
| **1** | 32µm SEBS/5µm TlE/**7**µ**m TPU**/5µm TIE/ 32µm SEBS | 25,0 |
| **2** | 30µm SEBS/5µm TlE/**10**µ**m TPU**/5µm TIE /30µm SEBS | 28,8 |
| **3** | 32µm (85%SEBS+15%SAB)/5µm TIE/ **7**µ**m TPU**/5µm TIE/32µm SEBS | 18,9 |
| **4** | 20µm SEBS/10µm EVA/5µm TIE/ **10**µ**m TPU**/5µm TIE/10µm EVA/20µm SEBS | 25,1 |
| **5** | 35µm EMA/**7**µ**m PA**/35µm EMA | 24,9 |
| **6** | 25µm EVA/**10**µ**m PVDC**/10µm LLDPE/ 30µm EVA | 16,6 |
| **7** | 15µm LLDPE/ **10**µ**m (95%COC+5%LLDPE)/** 15µm LLDPE | |
| **8** | 15µm LLDPE/ **10**µ**m (90%COC+10%**LLDPE)/ 15µm LLDPE | |

### EXAMPLE 8

Tensile strength at break (N/inch), machine direction (MD), has been tested according to ASTM D882 at 23°C and 50% RH.

**Table 8**

| | Test | Tensile Strength at break Machine direction (MD) |
|---|---|---|
| | Test method | ASTM D882 |
| | Test conditions | 23 °C, 50 % RH |
| | Unit | N / Inch |

| Film no. | Composition | |
|---|---|---|
| **1** | 32µm SEBS/5µm TIE/**7**µ**m TPU**/5µm TIE/ 32µm SEBS | 28,7 |
| **2** | 30µm SEBS/5µm TIE/**10**µ**m TPU**/5µm TIE /30µm SEBS | 34,7 |
| **3** | 32µm (85%SEBS+15%SAB)/5µm TIE/ **7**µ**m TPU**/5µm TIE/32µm SEBS | 27,5 |
| **4** | 20µm SEBS/10µm EVA/5µm TIE/ **10**µ**m TPU**/5µm TlE/10µm EVA/20µm SEBS | 26,2 |
| **5** | 35µm EMA/**7**µ**m PA**/35µm EMA | 32,5 |
| **6** | 25µm EVA/**10**µ**m PVDC**/10µm LLDPE/ 30µm EVA | 32,3 |
| **7** | 15µm LLDPE/ **10**µ**m (95%COC+5%LLDPE)/** 15µm LLDPE | |
| **8** | 15µm LLDPE/ **10**µ**m (90%COC+10%LLDPE)/** 15µm LLDPE | |

### EXAMPLE 9

Elongation at break (%), machine direction (MD) has been tested according to ASTM D882 at 23°C and 50% RH.

**Table 9**

| | Test | Elongation at break Machine direction (MD) |
|---|---|---|
| | Test method | ASTM D882 |
| | Test conditions | 23 °C, 50 % RH |
| | Unit | % |

| Film no. | Composition | |
|---|---|---|
| **1** | 32µm SEBS/5µm TIE/**7**µ**m TPU**/5µm TIE/ 32µm SEBS 200 | |
| **2** | 30µm SEBS/5µm TIE/**10**µ**m TPU**/5µm TIE /30µm SEBS | 175 |
| **3** | 32µm (85%SEBS+15%SAB)/5µm TIE/ **7**µ**m TPU**/5µm TIE/32µm SEBS | 185 |
| **4** | 20µm SEBS/10µm EVA/5µm TIE/ **10**µ**m TPU**/5µm TIE/10µm EVA/20µm SEBS | 186 |
| **5** | 35µm EMA/**7**µ**m PA**/35µm EMA | 169 |
| **6** | 25µm EVA/**10**µ**m PVDC**/10µm LLDPE/ 30µm EVA | 627 |
| **7** | 15µm LLDPE/ **10**µ**m(95%COC+5%LLDPE)/** 15µm LLDPE | |
| **8** | 15µm LLDPE/ **10**µ**m (90%COC+10%LLDPE)/** 15µm LLDPE | |

### EXAMPLE 10

Tensile strength at break (N/inch), transverse direction (TD) has been tested according to ASTM D882 at 23°C and 50% RH.

**Table 10**

| | Test | Tensile Strength at break Transverse direction (TD) |
|---|---|---|
| | Test method | ASTM D882 |
| | Test conditions | 23 °C, 50 % RH |
| | Unit | N/inch |

| Film no. | Composition | |
|---|---|---|
| **1** | 32µm SEBS/5µm TIE/**7**µ**m TPU**/5µm TIE/ 32µm SEBS | 23,7 |
| **2** | 30µm SEBS/5µm TIE/**10**µ**m TPU**/5µm TIE /30µm SEBS | 30,0 |
| 3 | 32µm (85%SEBS+15%SAB)/5µm TIE/ **7**µ**m TPU**/5µm TIE/32µm SEBS | 23,8 |
| **4** | 20µm SEBS/10µm EVA/5µm TIE/ **10**µ**m TPU**/5µm TIE/10µm EVA/20µm SEBS | 23,7 |
| **5** | 35µm EMA/**7**µ**m PA**/35µm EMA | 24,8 |
| **6** | 25µm EVA/**10**µ**m PVDC**/10µm LLDPE/ 30µm EVA | 26,2 |
| **7** | 15µm LLDPE/ **10**µ**m (95%COC+5%LLDPE)/** 15µm LLDPE | |
| **8** | 15µm LLDPE/ **10**µ**m (90%COC+10%LLDPE)/** 15µm LLDPE | |

### EXAMPLE 11

Elongation at break (%), transverse direction (TD), has been tested according to ASTM D882 at 23°C and 50% RH.

**Table 11**

| | Test | Elongation at break Transverse direction (TD) |
|---|---|---|
| | Test method | ASTM D882 |
| | Test conditions | 23 °C, 50 % RH |
| | Unit | % |

| Film no. | Composition | |
|---|---|---|
| **1** | 32µm SEBS/5µm TIE/**7**µ**m TPU**/5µm TIE/ 32µm SEBS | 266 |
| **2** | 30µm SEBS/5µm TlE/**10**µ**m TPU**/5µm TIE /30µm SEBS | 226 |
| **3** | 32µm (85%SEBS+15%SAB)/5µm TIE/ **7**µ**m TPU**/5µm TIE/32µm SEBS | 251 |
| **4** | 20µm SEBS/10µm EVA/5µm TIE/ **10**µ**m TPU**/5µm TIE/10µm EVA/20µm SEBS | 248 |
| **5** | 35µm EMA/**7**µ**m PA**/35µm EMA | 195 |
| **6** | 25µm EVA/**10**µ**m PVDC**/10µm LLDPE/ 30µm EVA | 818 |
| **7** | 15µm LLDPE/ **10**µ**m (95%COC+5%LLDPE)/** 15µm LLDPE | |
| **8** | 15µm LLDPE/ **10**µ**m (90%COC+10%LLDPE)/** 15µm LLDPE | |

### EXAMPLE 12

Puncture resistance at break (N) has been tested at 23°C and 50% RH and has been measured by pressing a round needle head diameter of 0.795 mm through a material at a constant speed. The force (N), which was necessary in order to press the needle through the material, has been registered. The test has been made out from a standard WI No. 261283.

A specimen of the flexible packaging material has been fastened in a sample holder. A probe (needle) has penetrated the specimen with a constant speed of 5 mm/min. The maximum force needed to penetrate the specimen has been recorded. The puncture resistance has been measured as the maximum force, which the material can withstand.

**Table 12**

| | Test | Puncture resistance at break |
|---|---|---|
| | Test method | See text |
| | Test conditions | 23 °C, 50 % RH Round Needle Head diameter = 0,795 mm |
| | Unit | N |

| Film no. | Composition | |
|---|---|---|
| **1** | 32µm SEBS/5µm TIE/**7**µ**m TPU**/5µm TIE/ 32µm SEBS | 2,1 |
| **2** | 30µm SEBS/5µm TIE/**10**µ**m TAU**/5µm TIE /30µm SEBS | 2,5 |
| **3** | 32µm (85%SEBS+15%SAB)/5µm TIE/ **7**µ**m TPU**/5µm TIE/32µm SEBS | 2,3 |
| **4** | 20µm SEBS/10µm EVA/5µm TIE/ **10**µ**m TPU**/5µm TIE/10µm EVA/20µm SEBS | 2,0 |
| **5** | 35µm EMA/**7**µ**m PA**/35µm EMA | 2,3 |
| **6** | 25µm EVA/**10**µ**m PVDC**/10µm LLDPE/ 30µm EVA | 1,5 |
| **7** | 15µm LLDPE/ **10**µ**m (95%COC+5%LLDPE)/** 15µm LLDPE | |
| **8** | 15µm LLDPE/ **10**µ**m (90%COC+10%LLDPE)/** 15µm LLDPE | |

### EXAMPLE 13

Skatole (3-Methyl Indole) break through time (hours) has been tested according to BS 7127, part 101 (1991) at 40 °C, and the REMARKS cited on page 3, lines 3-11 in this description.

**Table 13**

| | Test | Skatole (3-Methyl Indole) Break through time (BT) |
|---|---|---|
| | Test method | BS 7127, part 101 (1991) and REMARKS |
| | Test conditions | 40 °C |
| | Unit | hours |

| Film no. | Composition | |
|---|---|---|
| **1** | 32µm SEBS/5µm TIE/**7**µ**m TPU**/5µm TIE/ 32µm SEBS | > 24 |
| **2** | 30µm SEBS/5µm TIE/**10**µ**m TPU**/5µm TIE /30µm SEBS | > 24 |
| **3** | 32µm (85%SEBS+15%SAB)/5µm TIE/ **7**µ**m TPU**/5µm TIE/32µm SEBS | > 24 |
| **4** | 20µm SEBS/10µm EVA/5µm TIE/ **10**µ**m TPU**/5µm TIE/10µm EVA/20µm SEBS | > 24 |
| **5** | 35µm EMA/**7**µ**m PA**/35µm EMA | 5 -> 24 |
| **6** | 25µm EVA/**10**µ**m PVDC**/10µm LLDPE/ 30µm EVA | > 24 |
| **7** | 15µm LLDPE/ **10**µ**m (95%COC+5%LLDPE)/** 15µm LLDPE | > 24 |
| **8** | 15µm LLDPE/ **10**µ**m (90%COC+10%LLDPE)/** 15µm LLDPE | > 24 |

### EXAMPLE 14

Skatole (3-Methyl Indole) break through time (hours) has been tested according to BS 7127, part 101 (1991) at 40 °C and the REMARKS cited on page 3, lines 3-11 in this description after the film has been flexed 50 times.

The flexing of the film has been performed according to Federal Standard 101 C method 2017.

**Table 14**

| | Test | Skatole (3-Methyl Indole) Break through time (BT) after 50 flexings |
|---|---|---|
| | Test method | Skatole: BS 7127, part 101 (1991) and REMARKS Flexing: See text |
| | Test conditions | 40 °C |
| | Unit | hours |

| Film no. | Composition | |
|---|---|---|
| **1** | 32µm SEBS/5µm TIE/**7**µ**m TPU**/5µm TIE/ 32µm SEBS | > 24 |
| **2** | 30µm SEBS/5µm TIE/**10**µ**m TPU**/5µm TIE /30µm SEBS | > 24 |
| **3** | 32µm (85%SEBS+15%SAB)/5µm TIE/ **7**µ**m TPU**/5µm TIE/32µm SEBS | > 24 |
| **4** | 20µm SEBS/10µm EVA/5µm TIE/ **10**µ**m TPU**/5µm TIE/10µm EVA/20µm SEBS | > 24 |

### EXAMPLE 15

The skin layer of a multilayer film may be made of thermoplastic polyurethane having an E-modulus of less than 100 MPa or 15000 Psi. However, such soft thermoplastic polyurethane is not suited as an aroma barrier layer in a multilayer film according to the present invention. Therefore, a film of soft thermoplastic polyester-based polyurethane (Estane 58277 Nat. 021, Noveon) having an E-modulus 8.9 MPa at an elongation of 50% was tested. The film was extruded into a thickness of 65 µm.

This film of soft thermoplastic polyurethane was tested for a skatole (3-Methyl Indole) break through time (hours) according to BS 7127, part 101 (1991) at 40 °C and the REMARKS cited on page 3, lines 3-11 in this description. A skatole penetration was observed already after one hour - resulting in a break though time of less than 1 hour.

### EXAMPLE 16

The purpose of this test was to show the effect of the sulphur absorber. DMDS was tested instead of skatole using a concentration of 2,3g DMDS / L water.
The effect of having a sulphur absorber in the skin layer was tested using film no. 1, 3, and 6. The break through time (hours) of DMDS was tested in accordance with BS 7127, part 101 (1991) at 40 °C and the REMARKS cited on page 3, lines 3-11 in this description.

The test was carried out as a smell test, the decisive parameter thus being whether or not a person was able to smell DMDS. The score "1" indicates that no DMDS could be registered and the score "2" indicates a weak smell of DMDS. The number "3", "4", "5" indicates a still stronger smell of DMDS.

**Table 16**

| | 15 min | 30 min | 45 min | 75 min | 90 min | 120 min | 290 min |
|---|---|---|---|---|---|---|---|
| | | | | | | | |
| Film 6 | 1 | 1 | 1 | 1 | 1 | 2 | 3 |
| | | | | | | | |
| Film 1 | 1 | 1 | 1 | 2 | 3 | 3 | - |
| | | | | | | | |
| Film 3 | 1 | 1 | 1 | 1 | 1 | 1 | 1 |

As can be seen from the test results in the above-mentioned examples, thermoplastic polyurethane as an aroma barrier film is an excellent alternative to known halogen-free polymer films since thermoplastic polyurethane as a barrier layer in a multilayer film is better than a barrier layer of polyamide in regard to skatole.

In example 16, a multilayer film having a barrier layer of 7 µm TPU as film 1 was compared with the same film having a sulphur absorber in the skin layer furthest from the aroma compound as film 3. As can be seen, film 1 without sulphur absorber has a quicker penetration of DMDS than the same film with sulphur absorber namely film 3, and film 3 even has a better aroma barrier to DMDS than film 6 containing halogen, PVdC

The tests have also shown that thermoplastic polyurethane as a barrier layer in a multilayer film is better than PVdC in regards to puncture resistance and seal strength.

Furthermore, as can be seen from the test results presented in Table 14, TPU as a barrier layer has a very good flex crack resistance.

Therefore, a multilayer film free of halogens which is impermeable to aroma compounds and has an aroma barrier layer comprising thermoplastic polyurethane polymer has been found to be a good alternative to the known multilayer films in particular useful for the production of pouches, such as ostomy pouches and drainable bags.

Also, cyclic polyolefin (COC) has been found as a good alternative as a barrier layer in the above-mentioned multilayer film, since cyclic polyolefin as a barrier layer is better than a barrier layer of polyamide and equally good as a barrier layer of PVdC in regard to skatole.

The oxygen permeability test has also shown that a barrier layer of TPU or COC is not as good as a barrier layer of PA or PVdC. However, O₂ does not smell and the oxygen permeability test results are therefore not important when the film is used for the production of pouches, such as ostomy pouches and drainable bags.

## Claims

1. A multilayer film (1) free of halogens which is impermeable to aroma compounds, wherein a multilayer film impermeable to aroma compounds is defined as a multilayer film having an aroma barrier layer with a thickness of 15 µm or less being impermeable to skatole for at least four hours when subjected to a skatole-test performed according a British standard, BS 7127, part 101 (1991) at 40 °C, comprising
- at least one aroma barrier layer (3; 15), and
- at least one sealable skin layer (2, 4; 12, 14)
**characterised in that** the aroma barrier layer comprises more than 40 weight% of thermoplastic polyurethane polymer
having an E-modulus of at least 600 MPa, at least 1000 MPa, at least 1700 MPa, or at least 2000 MPa.

2. A multilayer film as claimed in claim 1, wherein the multilayer film further comprises a sulphur absorber and wherein the sulphur absorber is comprised in the skin layer.

3. A multilayer film as claimed in any of the preceding claims, wherein the aroma barrier layer (3; 15) has a thickness of 1 - 50 µm, 2 - 25 µm, or 3 - 10 µm.

4. A multilayer film as claimed in any of the preceding claims, wherein the skin layer has an E-modulus that is less than 100 MPa or 15,000 Psi.

5. A multilayer film as claimed in any of the preceding claims, wherein the skin layer (2, 4; 12, 14) comprises:
- styrene-based copolymers, such as styrene ethylene butylene styrene copolymer (SEBS), styrene ethylene propylene styrene copolymer (SEPS), styrene butadiene styrene copolymer (SBS), or styrene isoprene styrene copolymer (SIS):
- ethylene-based polymers, such as pure polyethylene in the form of low density polyethylene (LDPE), linear low density polyethylene (LLDPE), ultra low density polyethylene (LLDPE), medium density polyethylene (MDPE), high density polyethylene (HDPE), or cyclic polyolefin (COC);
- ethylene-based co-polymers, such as ethylene methyl acrylate copolymer (EMA), ethylene vinyl acetate copolymer (EVA), ethylene butyl acrylate copolymer (EBA), othylene-ethyl acrylate (EEA), ethylene acrylic acid copolymer (EAA), ionomer resins, elastomeric oo-polyeaters, ethylene-methyl acrylic acid copolymers (EMAA), EVA-carbon monoxide copolymers (EVACO), MAH-modified polyethylene, maleic anhydride modified EVA, MAH-EMA MAH-EBA, MAH-PP, glycidyl methacrylate modified EMA, glycidyl methacrylate modified EBA, glycidyl methacrylate modified EVA, ethylene butylene copolymer, ethylene 4-methyl pentene copolymer, ethylene hexene co-polymer, ethylene octane copolymer, ethylene propylene copolymer, or ethylene butylene propylene ter-polymer;
- pure polypropylene, pure polybutylene, thermoplastic polyurethane (TPU), homogeneous EAO copolymers, PP homo- or copolymers, rubber modified PP, low modulus PP homo- or copolymers, low crystallinity PP homo- or copolymers, syndiotactic PP homo- or copolymers, ethylene-propylene-dlene monomer elastomer (EPDM), ethylene-polyporopylens rubbers (EPR), substantially linear EAO copolymers, ethylene-alkyl acrylate copolymers, such as, for example, polynorbornene, ESI, polyether-amlde block copolymers, polyamide (PA) or polyester (PETP): or
- any kind of blends of the above.

6. A multilayer film as claimed in any of the preceding claims, wherein the skin layer is embossed, texturised, foamed, non-woven, has been subjected to a finishing treatment such as pulling of threads, or has a silky surface.

7. A multilayer film as claimed in any of the preceding claims, wherein the aroma barrier layer (3; 15) is positioned in between two skin layers (2, 4; 12, 14).

8. A multilayer film as claimed in claim 7, wherein the sulphur absorber is comprised in the skin layer positioned furthest from the aroma compounds so that the aroma compounds have to enter the barrier layer before arriving at the skin layer containing sulphur absorber.

9. A multilayer film as claimed in any of the preceding claims, wherein the skin layer (2, 4; 12, 14) has a thickness of 5 - 150 µm, 10 -100 µm, or 15 - 60 µm.

10. A multilayer film as claimed in any of the preceding claims, further comprising an additional layer (13) positioned between the skin layer (12, 14) and the aroma barrier layer (15).

11. A multilayer film as claimed in claim 10, wherein the additional layer is a tie layer comprising:
- styrene-based copolymers, such as styrene ethylene butylene styrene copolymer (SEBS), styrene ethylene propylene styrene copolymer (SEPS), styrene butadiene styrene copolymer (SBS), or styrene isopneae styrene copolymer (SIS);
- ethylene-based polymers, such as pure polyethylene in the form of low density polyethylene (LDPE), linear tow density polyethylene (LLDPE), ultra low density poly-ethylene (ULDPE), medium density polyethylene (MDPE), high density polyethylene (HDPE), or cyclic polyolefin (COC);
- ethytene-based co-polymers, such as ethylene methyl acrylate copolymer (EMA), ethylene vinyl acetate copolymer (EVA), ethylene butyl acrylate copolymer (ESA), ethylene-ethyl acrylate (EEA), ethylene acrylic add copolymer (EAA), ionomer resins, elastomeric co-polyesters, ethpens-methyl acrylic acid copolymers (EMAA). EVA-carbon monoxide copolymers (EVACO), MAH-modifiad polyethylene, maleic anhydride modified EVA, MAH-EMA, MAH-EBA, MAH-PP, glycidyl methacrylate modified EMA, glycidyl methacrylate modified EBA, glycidyl methacrylate modified EVA, ethylene butylene copolymer, ethylene 4-methyl pentene copolymer, ethylene hexene copolymer; ethylene octene copolymer, ethylene propylene copolymer, or ethylene butylene propylene ter-polymer;
- pure polypropylene, pure polybutylene, thermoplastic polyurethane (TPU), homogeneous EAO copolymers. PP homo- or copolymers, rubber modified. PP. low modulus PP homo- or copolymers, low crystallinity PP homo- or copolymers, syndiotactic. PP homo- or copolymers, ethylene-pmpylene-diene monomer elastomer (EPDM), ethlene-polypropylene rubbers (EPR), substantially linear EAO copolymers, ethylene-alkyl acrylate copolymers, such as, for example, polynorbornene, ESI, polyether-amlde block copolymers, polyamide (PA) or polyester (PETP); or
- any kind of blends of the above.

12. A multilayer film as claimed in claim 10, wherein the additional layer is an adhesive layer, such as a one-component or two-component polyurethane adhesive, a hotmelt, a wax, or the like.

13. A pouch, such as an ostomy pouch made of a multilayer film according to any of the preceding claims.

## Patentansprüche

1. Für Aromaverbindungen undurchlässige halogenfreie Mehrschichtfolie (1), wobei eine für Aromaverbindungen undurchlässige Mehrschichtfolie definiert ist als eine Mehrschichtfolie mit einer Aromasperrschicht mit einer Dicke von 15 µm oder weniger, die wenigstens vier Stunden lang für Skatol undurchlässig ist, wenn sie einer Skatolprüfung nach dem britischem Standard BS 7127, Teil 101 (1991), bei 40° unterzogen wird, umfassend:
- wenigstens eine Aromasperrschicht (3; 15), und
- wenigstens eine versiegelbare Außenschicht (2, 4; 12, 14)
**dadurch gekennzeichnet, daß** die Aromasperrschicht mehr als 40 Gewichtsprozent thermoplastisches Polyurethanpolymer mit einem E- Modul von wenigstens 600 MPa, wenigstens 1000 MPa, wenigstens 1700 MPa oder wenigstens 2000 MPa aufweist.

2. Mehrschichtfolie nach Anspruch 1, wobei die Mehrschichtfolie ferner einen Schwefelabsorber umfasst und wobei der Schwefelabsorber in der Außenschicht enthalten ist.

3. Mehrschichtfolie nach einem der vorhergehenden Ansprüche, wobei die Aromasperrschicht (3; 15) eine Dicke von 1 - 50 µm, 2 - 25 µm oder 3 - 10 µm hat.

4. Mehrschichtfolie nach einem der vorhergehenden Ansprüche, wobei die Außenschicht einen E- Modul von weniger als 100 MPa oder 15000 Psi hat.

5. Mehrschichtfolie nach einem der vorhergehenden Ansprüche, wobei die Außenschicht (2, 4; 12, 14) umfasst:
- auf Styrol basierende Copolymere, wie Styrol-Ethylen-Butylen-Styrol-Copolymer (SEBS), Styrol- Ethylen-Propylen-Styrol-Copolymer (SEPS), Styrol-Butadien-Styrol-Copolymer (SBS) oder Styrol-Isopren-Styrol-Copolymer (SIS);
- auf Ethylen basierende Polymere, wie reines Polyethylen in der Form von Polyethylen mit niedriger Dichte (LDPE), linearem Polyethylen mit niedriger Dichte (LLDPE), Polyethylen mit ultraniedriger Dichte (ULDPE), Polyethylen mit mittlerer Dichte (MDPE), Polyethylen mit hoher Dichte (HDPE) oder cyklisches Polyolefin (COC);
- auf Ethylen basierende Copolymere, wie etwa Ethylen-Methylacrylat-Copolymer (EMA), Ethylen-Vinylacetat-Copolymer (EVA), Ethylen-Butylacrylat-Copolymer (EBA), Ethylen-Ethylacrylat (EEA), Ethylen-Acrylsäure-Copolymer (EAA), Ionomerharze, elastomere Copolyester, Ethylen-Methylacrylsäure-Copolymere (EMAA), EVA-Kohlenmonoxid-Copolymere (EVACO), MAH-modifziertes Polyethylen, Maleinsäureanhydrid-modifiziertes EVA, MAH-EMA, MAH-EBA, MAH-PP, Glycidylmethacrylat-modifiziertes EMA, Glycidylmethacrylat-modifiziertes EBA, Glycidylmethacrylat-modifiziertes EVA, Ethylen-Butylen-Copolymer, Ethylen-4-Methylpenten-Copolymer, Ethylen-Hexen-Copolymer, Ethylen-Octen-Copolymer, Ethylen-Propylen-Copolymer oder Ethylen-Butylen-Propylen-Terpolymer;
- reines Polypropylen, reines Polybutylen, thermoplastisches Polyurethan (TPU), homogene EAO-Copolymere, PP-Homo- oder Copolymere, Kautschuk modifiziertes PP, PP-Homo- oder Copolymere mit niedrigem Modulus, PP-Homo- oder Copolymere mit geringer Kristallinität, syndiotaktische PP-Homo- oder Copolymere, Ethylen-Propylen-Dien-Monomer-Elastomer (EPDM), Ethylen-Polypropylen-Kautschuke (EPR), im wesentlichen lineare EAO-Copolymere, Ethylen-Alkylacrylat-Copolymere, wie zum Beispiel Polynorbornen, ESI, Polyether-Amid-Blockcopolymere, Polyamid (PA) oder Polyester (PETP); oder
- jede Art von Mischungen der Vorstehenden.

6. Mehrschichtfolie nach einem der vorhergehenden Ansprüche, wobei die Außenschicht geprägt, texturiert, geschäumt, nicht gewebt ist, einer Endbehandlung, wie Ziehen von Fäden, unterzogen wurde oder eine seidige Oberfläche hat.

7. Mehrschichtfolie nach einem der vorhergehenden Ansprüche, wobei die Aromasperrschicht (3; 15) zwischen zwei Außenschichten (2, 4; 12, 14) angeordnet ist.

8. Mehrschichtfolie nach Anspruch 7, wobei der Schwefelabsorber in der Außenschicht enthalten ist, die am weitesten von den Aromaverbindungen entfernt ist, so daß Aromaverbindungen in die Sperrschicht eintreten müssen, bevor sie an der Außenschicht, die den Schwefelabsorber enthält, ankommen.

9. Mehrschichtfolie nach einem der vorhergehenden Ansprüche, wobei die Außenschicht (2, 4; 12, 14) eine Dicke von 5 - 150 µm, 10 - 100 µm oder 15 - 50 µm hat.

10. Mehrschichtfolie nach einem der vorhergehenden Ansprüche, die ferner eine Zusatzschicht (13) umfasst, die zwischen der Außenschicht (12, 14) und der Aromasperrschicht (15) angeordnet ist.

11. Mehrschichtfolie nach Anspruch 10, wobei die Zusatzschicht eine Verbindungsschicht ist, umfassend:
- auf Styrol basierende Copolymere, wie Styrol-Ethylen-Butylen-Styrol-Copolymer (SEBS), Styrol-Ethylen-Propylen-Styrol-Copolymer (SEPS), Styrol-Butadien-Styrol-Copolymer (SBS) oder Styrol-Isopren-Styrol-Copolymer (SIS);
- auf Ethylen basierende Polymere, wie reines Polyethylen in der Form von Polyethylen mit niedriger Dichte (LDPE), linearem Polyethylen mit niedriger Dichte (LLDPE), Polyethylen mit ultraniedriger Dichte (ULDPE), Polyethylen mit mittlerer Dichte (MDPE), Polyethylen mit hoher Dichte (HDPE) oder cyklisches Polyolefin (COC);
- auf Ethylen basierende Copolymere, wie Ethylen-Methylacrylat-Copolymer (EMA), Ethylen-Vinylacetat-Copolymer (EVA), Ethylen-Butylacrylat-Copolymer (EBA), Ethylen-Ethylacrylat (EEA), Ethylen-Acrylsäure-Copolymer (EAA), Ionomerharze, elastomere Copolyester, Ethylen-Methylacrylsäure-Copolymere (EMAA), EVA-Kohlenmonoxid-Copolymere (EVACO), MAH-modifziertes Polyethylen, Maleinsäureanhydrid-modifiziertes EVA, MAH-EMA, MAH-EBA, MAH-PP, Glycidylmethacrylat-modifiziertes EMA, Glycidylmethacrylat-modifiziertes EBA, Glycidylmethacrylat-modifiziertes EVA, Ethylen-Butylen-Copolymer, Ethylen-4-Methylpenten-Copolymer, Ethylen-Hexen-Copolymer, Ethylen-Octen-Copolymer, Ethylen-Propylen-Copolymer oder Ethylen-Butylen-Propylen-Terpolymer;
- reines Polypropylen, reines Polybutylen, thermoplastisches Polyurethan (TPU), homogene EAO-Copolymere, PP-Homo- oder Copolymere, Kautschuk modifiziertes PP, PP-Homo- oder Copolymere mit niedrigem Modulus, PP-Homo- oder Copolymere mit geringer Kristallinität, syndiotaktische PP-Homo- oder Copolymere, Ethylen-Propylen-Dien-Monomer-Elastomer (EPDM), Ethylen-Polypropylen-Kautschuke (EPR), im wesentlichen lineare EAO-Copolymere, Ethylen-Alkylacrylat-Copolymere, wie zum Beispiel Polynorbornen, ESI, Polyether-Amid-Blockcopolymere, Polyamid (PA) oder Polyester (PETP); oder
- jede Art von Mischungen der Vorstehenden.

12. Mehrschichtfolie nach Anspruch 10, wobei die Zusatzschicht eine Klebstoffschicht, wie ein Einkomponenten- oder Zweikomponenten-Polyurethanklebstoff, ein Heißschmelzkleber, ein Wachs oder ähnliches, ist.

13. Beutel, wie ein Ostomiebeutel, gefertigt aus einer Mehrschichtfolie nach einem der vorhergehenden Ansprüche.

## Revendications

1. Film multicouche (1) exempt d'halogènes, qui est imperméable aux composés de type arômes, où un film multicouche imperméable aux composés de type arômes est défini comme étant un film multicouche ayant une couche formant barrière aux arômes, ayant une épaisseur de 15 µm ou moins et imperméable au scatole pendant au moins 4 h lorsqu'il est soumis à un essai au scatole réalisé selon une norme britannique, BS 7127, partie 101 (1991) à 40°C, comprenant
- au moins une couche formant barrière aux arômes (3 ; 15), et
- au moins une couche pelliculaire scellable (2, 4 ; 12, 14) **caractérisé en ce que** la couche formant barrière aux arômes comprend plus de 40 % en poids de polymère polyuréthane thermoplastique ayant un module E d'au moins 600 MPa, d'au moins 1 000 MPa, d'au moins 1 700 MPa ou d'au moins 2 000 MPa.

2. Film multicouche selon la revendication 1, où le film multicouche comprend en outre une substance absorbant le soufre et où la substance absorbant le soufre est contenue dans la couche pelliculaire.

3. Film multicouche selon l'une quelconque des revendications précédentes, où la couche formant barrière aux arômes (3 ; 15) a une épaisseur de 1 à 50 µm, 2 à 25 µm ou 3 à 10 µm,

4. Film multicouche selon l'une quelconque des revendications précédentes, où la couche pelliculaire a un module E inférieur à 100 MPa ou 15 000 Psi.

5. Film multicouche selon l'une quelconque des revendications précédentes, où la couche pelliculaire (2, 4 ; 12, 14) comprend :
- des copolymères à base de styrène, tels que copolymère styrène-éthylène-butylène-styrène (SEBS), copolymère styrène-éthylène-propylène-styrène (SEPS), copolymère styrène-butadiène-styrène (SBS) ou copolymère styrène-isoprène-styrène (SIS) ;
- des polymères à base d'éthylène, tels que polyéthylène pur sous forme de polyéthylène basse densité (LDPE), polyéthylène linéaire basse densité (LLDPE), polyéthylène ultra basse densité (ULDPE), polyéthylène densité moyenne (MDPE), polyéthylène haute densité (HDPE) ou polyoléfine cyclique (COC) ;
- des copolymères à base d'éthylène, tels que copolymère éthylène-acrylate de méthyle (EMA), copolymère éthylène-acétate de vinyle (EVA), copolymère éthylène-acrylate de butyle (EBA), copolymère éthylène-acrylate d'éthyle (EEA), copolymère éthylène-acide acrylique (EAA), résines ionomères, copolyesters élastomères, copolymères éthylène-acide méthylacrylique (EMAA), copolymères EVA-monoxyde de carbone (EVACO), polyéthylène modifié par MAH, EVA modifié par l'anhydride maléique, MAH-EMA, MAH-EBA, MAH-PP, EMA modifié par le méthacrylate de glycidyle, EBA modifié par le méthacrylate de glycidyle, EVA modifié par le méthacrylate de glycidyle, copolymère éthylène-butylène, copolymère éthylène-4-méthylpentène, copolymère éthylène-hexène, copolymère éthylène-octène, copolymère éthylène-propylène ou terpolymère éthylène-butylène-propylène ;
- du polypropylène pur, du polybutylène pur, du polyuréthane thermoplastique (TPU), des copolymères EAO homogènes, des homo- ou copolymères PP, PP modifié par le caoutchouc, des homo- ou copolymères PP de bas module, des homo- ou copolymères PP de basse cristallinité, des homo- ou copolymères PP syndiotactiques, l'élastomère monomère éthylène-propylène-diène (EPDM), des caoutchoucs éthylène-polypropylène (EPR), des copolymères EAO sensiblement linéaires, des copolymères éthylène-acrylate d'alkyle, tels que par exemple polynorbornène, ESI, des copolymères blocs polyéther-amide, du polyamide (PA) ou du polyester (PETP) ; ou
- tout type de mélange des éléments ci-dessus.

6. Film multicouche selon l'une quelconque des revendications précédentes, où la couche pelliculaire est gaufrée, texturée, moussée, non tissée, a été soumise à un traitement de finition tel que tirage de fils ou a une surface soyeuse.

7. Film multicouche selon l'une quelconque des revendications précédentes, où la couche formant barrière aux arômes (3 ; 15) est positionnée entre deux couches pelliculaires (2, 4 ; 12, 14).

8. Film multicouche selon la revendication 7, où la substance absorbant le soufre se trouve positionnée dans la couche pelliculaire aussi loin que possible des composés de type arômes de telle sorte que les composés de type arômes doivent traverser la couche formant barrière avant d'arriver à la couche pelliculaire contenant la substance absorbant le soufre.

9. Film multicouche selon l'une quelconque des revendications précédentes, où la couche pelliculaire (2, 4 ; 12, 14) a une épaisseur de 5 à 150 µm, 10 à 100 µm ou 15 à 60 µm,

10. Film multicouche selon l'une quelconque des revendications précédentes, comprenant en outre une couche additionnelle (13) positionnée entre la couche pelliculaire (12, 14) et la couche formant barrière aux arômes (15).

11. Film multicouche selon la revendication 10, où la couche additionnelle est une couche de liaison comprenant :
- des copolymères à base de styrène, tels que copolymère styrène-éthylène-butylène-styrène (SEBS), copolymère styrène-éthylène-propylène-styrène (SEPS), copolymère styrène-butadiène-styrène (SBS) ou copolymère styrène-isoprène-styrène (SIS) ;
- des polymères à base d'éthylène, tels que polyéthylène pur sous la forme de polyéthylène basse densité (LDPE), polyéthylène linéaire basse densité (LLDPE), polyéthylène ultra basse densité (ULDPE), polyéthylène densité moyenne (MDPE), polyéthylène haute densité (HDPE) ou polyoléfine cyclique (COC) ;
- des copolymères à base d'éthylène, tels que copolymère éthylène-acrylate de méthyle (EMA), copolymère éthylène-acétate de vinyle (EVA), copolymère éthylène-acrylate de butyle (EBA), copolymère éthylène-acrylate d'éthyle (EEA), copolymère éthylène-acide acrylique (EAA), résines ionomères, copolyesters élastomères, copolymères éthylène-acide méthylacrylique (EMAA), copolymères EVA-monoxyde de carbone (EVACO), polyéthylène modifié par MAH, EVA modifié par l'anhydride maléique, MAH-EMA, MAH-EBA, MAH-PP, EMA modifié par le méthacrylate de glycidyle, EBA modifié par le méthacrylate de glycidyle, EVA modifié par le méthacrylate de glycidyle, copolymère éthylène-butylène, copolymère éthylène-4-méthylpentène, copolymère éthylène-hexène, copolymère éthylène-octène, copolymère éthylène-propylène ou terpolymère éthylène-butylène-propylène ;
- du polypropylène pur, du polybutylène pur, du polyuréthane thermoplastique (TPU), des copolymères EAO homogènes, des homo- ou copolymères PP, PP modifié par le caoutchouc, des homo- ou copolymères PP de bas module, des homo- ou copolymères PP de basse cristallinité, des homo- ou copolymères PP syndiotactiques, l'élastomère monomère éthylène-propylène-diène (EPDM), des caoutchoucs éthylène-polypropylène (EPR), des copolymères EAO sensiblement linéaires, des copolymères éthylène-acrylate d'alkyle, tels que par exemple polynorbornène, ESI, des copolymères blocs polyéther-amide, du polyamide (PA) ou du polyester (PETP) ; ou
- tout type de mélange des éléments ci-dessus.

12. Film multicouche selon la revendication 10, où la couche additionnelle est une couche adhésive, par exemple un adhésif de polyuréthane à un ou deux composants, une colle thermofusible autoadhésive, une cire ou similaire.

13. Poche, telle que poche pour anus artificiel, faite d'un film multicouche selon l'une quelconque des revendications précédentes.
